(19) Europäisches Patentamt
European Patent Office
Office européen des brevets

(11) Publication number: **0 664 284 A1**

(12) # EUROPEAN PATENT APPLICATION

(21) Application number: **95100292.2**

(22) Date of filing: **11.01.95**

(51) Int. Cl.[6]: **C07C 249/08**, C07C 237/22, C07C 69/40

(30) Priority: **21.01.94 EP 94100852**

(43) Date of publication of application: **26.07.95 Bulletin 95/30**

(84) Designated Contracting States: **AT BE CH DE DK ES FR GB IT LI NL**

(71) Applicant: **F. HOFFMANN-LA ROCHE AG**
**Postfach 3255**
**CH-4002 Basel (CH)**

(72) Inventor: **Soukup, Milan Dr.**
**Efeuweg 5**
**CH-4102 Bottmingen (CH)**
Inventor: **Wirz, Beat**
**4 Wiedenweg**
**CH-4153 Reinach (CH)**

(74) Representative: **Mahé, Jean et al**
**Postfach 3255**
**Grenzacherstrasse 124**
**CH-4002 Basel (CH)**

(54) **Process for preparing carboxamides.**

(57) The invention relates to a novel process for the preparation of hydroxyamic acid derivatives of the formula

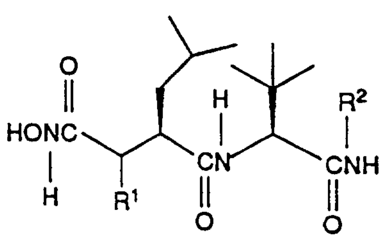

I

wherein $R^1$ and $R^2$ are as defined in the specification, via the corresponding novel carboxylic acid esters, and to novel intermediates occurring in this process.

EP 0 664 284 A1

The present invention is concerned with a novel process for the manufacture of carboxamides of the formula I:

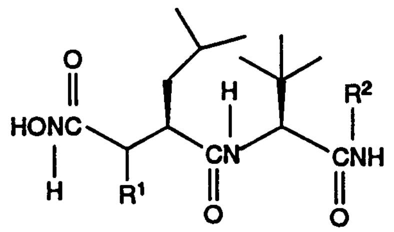

I

wherein

- $R^1$ is H, protected amino, acylamino or lower alkyl optionally substituted by aryl, protected hydroxy, protected amino, acylamino, succinimido, 2,3-dihydro-1,3-dioxo-1H-benz[d,e]isoquinol-2-yl, protected carboxy, carbamoyl, carboxy-lower alkanoylamino, pyrrolidino or morpholino, and
- $R^2$ is lower alkyl optionally substituted by protected carboxy or by carbamoyl, mono or di(lower alkyl)carbamoyl or morpholino,

which process comprises reacting a compound of the formula II:

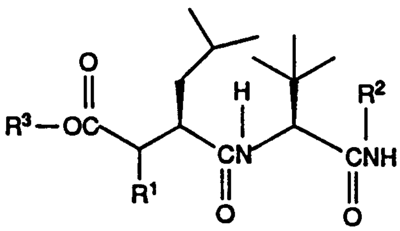

II

wherein $R^3$ is lower alkyl or aryl-lower alkyl, provided $R^3$ is not of the tert.butyl or neopentyl type, with a compound of the formula $H_2NO$-M, wherein M is an alkali metal.

Compounds of formula I are known, e.g. from EP 497 192 A2. They possess valuable pharmacological properties and can be used for the control or prevention of illnesses.

As used in this Specification, alone or in combination, the term "lower alkyl" means a straight-chain or branched-chain alkyl group containing a maximum of six carbon atoms, such as methyl, ethyl, n-propyl, isopropyl, n-butyl, sec.butyl, isobutyl, tert.butyl, n-pentyl, n-hexyl and the like. Likewise, the term "lower alkoxy" means a straight-chain or branched-chain alkoxy group containing a maximum of six carbon atoms, such as methoxy, ethoxy, n-propoxy, isopropoxy, n-butoxy, tert.butoxy and the like. The term "aryl" means an optionally substituted phenyl or naphthyl group with the substituent(s) being selected, for example, from halogen, trifluoromethyl, lower alkyl, lower alkoxy, phenyl and the like. The acyl part of an acylamino group is derived from an alkanoic acid which contains a maximum of six carbon atoms, e.g. acetyl, propionyl, butyryl, pivaloyl etc, from an optionally substituted benzoic or naphthoic acid, e.g. benzoyl, 4-chlorobenzoyl, 2-carboxybenzoyl, 1- or 2-naphthoyl etc, or from an arylsubstituted alkanoic acid which contains a maximum of six carbon atoms, e.g. phenylacetyl etc. The lower alkanoyl part of a carboxy-lower alkanoylamino group is derived from an alkanoic acid which contains a maximum of six carbon atoms, e.g. acetyl, propionyl, butyryl etc. The term "halogen" means fluorine, chlorine, bromine or iodine.

The terms "protected amino", "protected hydroxy" and "protected carboxy" mean amino, hydroxy and carboxy groups, respectively, which are protected in a manner known per se, e.g. as known in peptide chemistry. For example, an amino group can be protected by a benzyloxycarbonyl, tert.butoxycarbonyl, acetyl or like group or in the form of a phthalimido or like group. A hydroxy group can be protected, for example, in the form of a readily cleavable ether such as the tert.butyl or benzyl ether or in the form of a readily cleavable ester such as the acetate. Again, for example, a carboxy group can be protected in the form of a readily cleavable ester such as the methyl, ethyl, benzyl or like ester.

Examples of esters of formula II which can react with the alkali metal salts of formula $H_2NO$-M, are those wherein $R^3$ is propyl, n-butyl, benzyl and preferably ethyl. Examples of alkali metals M are Li, Na and preferably K.

Preferably, the hydroxylamine salt $H_2NO$-M is prepared in situ by reacting a solution of 2 equivalents of M-OH in water or in an other protic solvent, such as an alkanol, e.g. ethanol, an ether, such as THF, dioxane or diglyme, or DMSO, DMF or the like, with a salt, preferably the hydrochloride, of hydroxylamine. The obtained solution of $H_2NO$-M is then reacted with an ester of formula II preferably in water or in a solvent miscible with water, such as THF, at a temperature between -20 °C and 50 °C, preferably at about 0 °C.

The esters of formula II can be prepared by esterifying in a manner known per se the corresponding acids with an alcohol $R^3$-OH. These acids are described in the EP 497 192 A2.

In a further aspect of the invention the esters of formula II-A:

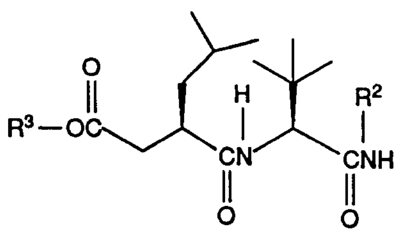

II-A

can also be prepared by reacting an acid halide or a mixed anhydride of the formula VI:

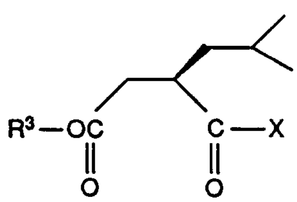

VI

wherein X is halogen, acyloxy or lower alkoxycarbonyloxy, with an amine of the formula VII:

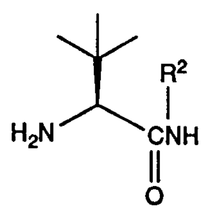

VII

wherein $R^2$ is as above.

This reaction can be performed in an aprotic solvent, such as hexane, toluene, ethyl acetate, diisopropylether, tert.butyl-methylether or acetonitrile, in the presence of a base, e.g. triethylamine, diisopropylethylamine, pyridine, dimethylaminopyridine, or Na or K carbonate or bicarbonate, at a temperature between 0 °C and 50 °C, preferably at about 15 °C.

The acid halides of formula VI can be obtained from the corresponding acids of the formula III:

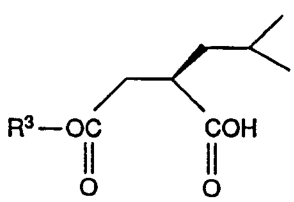

III

by reaction with an halogenating agent. Preferred acid halides are the acid chlorides. They can be prepared e.g. by reacting the acid with sulfurylchloride, oxalylchloride, $POCl_3$, $PCl_5$ or preferably thionylchloride. The chlorination is conveniently carried out in an apolar solvent such as hexane, toluene, acetonitrile or

preferably ethylacetate. A mixed anhydride VI, wherein X stands for acyloxy or alkoxycarbonyloxy, can be prepared by reacting the corresponding acid with an acylchloride, e.g. pivaloylchloride, or an alkoxycarbonylchloride, e.g. methylchloroformate.

The amides of formula VII are known compounds or analogues of known compounds which can be prepared in a manner known per se starting from the corresponding $\alpha$-aminoacid.

In a further aspect of the invention the acids of the above formula III are prepared by regioselective and enantioselective enzymatic hydrolysis of a racemic diester of the formula IV:

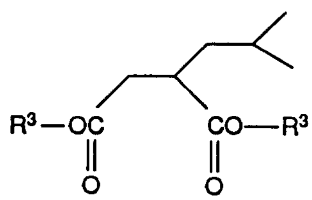

IV

This enzymatic hydrolysis is preferably carried out at a pH of 7.5 to 11.0, preferably at pH 8-10, in an aqueous emulsion of the diester IV. As enzymes there can be utilized preparation of subtilisin Carlsberg, i.e. a protease of the serine type produced from Bacillus licheniformis. Examples of such enzymes are Optimase M440, Protease L660, Alcalase 2,0T, Alcalase 2,5L and Subtilisin A. In the enzymatic hydrolysis of the racemic diester VI to the chiral acid III, there is also obtained the enantiomeric diester of the formula VIII:

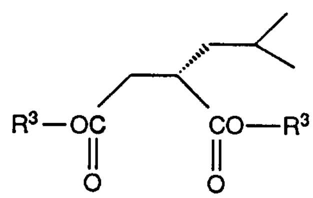

VIII

wherein $R^3$ is as above.

The separation of III from VIII can be carried out by basic/acid extraction. In a preferred form of the present invention the enantiomeric diester VIII can easily be recycled by racemization back to the racemic precursor IV, e.g. by distillation in the presence of a catalytic amount of sodium ethanolate or of an other analogous base.

In still a further aspect of the invention the diester of the formula IV can be prepared by a) reacting maleic anhydride with isobutylene, b) hydrogenating the olefinic bound in the obtained compound of the formula

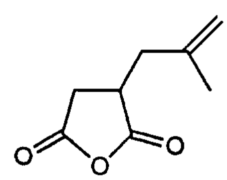

5

and c) reacting the obtained isobutylsuccinic anhydride with an alcohol of the formula $R^3$-OH.

The reaction of maleic anhydride with isobutylene can be carried out at a temperature between 150 and 250 °C, preferably at about 190-200 °C in the presence of hydroquinone. The use of a solvent, e.g. an apolar solvent such as toluene, while not critical, is preferred. The hydrogenation of the compound 5 is conveniently carried out by catalysis, e.g. with Pd/C, Pt or Raney-nickel, in an apolar solvent, such as hexane, toluene or ethylacetate. The reaction of isobutylsuccinic anhydride with an alcohol $R^3$-OH can be carried out in the presence of an acid commonly used in such esterification, e.g. p-toluenesulfonic acid or a Lewis acid, or in the presence of thionyl chloride. The temperature and the use of a solvent are not critical. However, the esterification is conveniently carried out at about 50-100 °C.

In a preferred embodiment of the invention a compound of the formula I-A is prepared by a) converting an acid of the formula III:

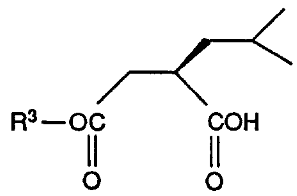

III

into an acid halogenide or into a mixed anhydride of the formula VI:

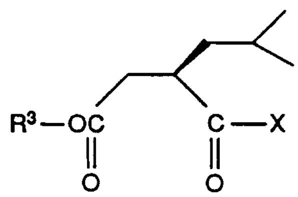

VI

b) reacting the compound of formula VI with an amine of the formula VII:

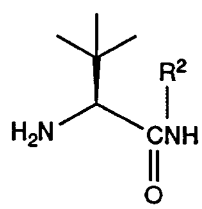

VII

and c) reacting the obtained compound of the formula II-A:

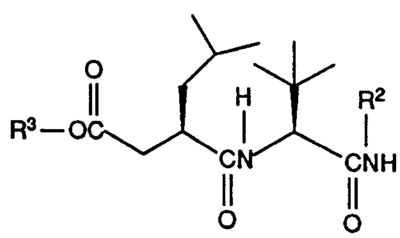

II-A

with a salt of the formula $H_2NO$-M, wherein M is an alkali metal.

In a further preferred embodiment, an acid of the formula III is prepared by a) reacting maleic anhydride with isobutylene, b) hydrogenating the olefinic bound in the obtained compound of the formula 5:

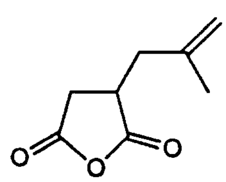

5

c) reacting the obtained isobutyl succinic anhydride with an alcohol of the formula $R^3$-OH, and d) enzymatically hydrolyzing the obtained diester of the formula IV:

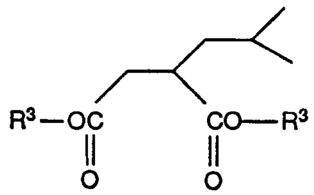

IV

The compounds of the formulae II (specially II-A), VI and VIII are novel and as such represent a further aspect of the invention. Examples of such compounds are:

N-2-[2(R)-(ethoxycarbonylmethyl)-4-methylvaleryl]-N-1,3-dimethyl-L-valinamide,
2(R)-4-methyl-2-(ethoxycarbonylmethyl)valeric acid chloride, and
(S)-diethyl-isobutylsuccinate.

Example 1

In a 20 l autoclave were added 1 kg maleic anhydride, 1.5 l toluene and 13 g hydroquinone. The autoclave was closed and loaded with 1.7 kg isobutylene and the reaction mixture was heated for 16 hrs at 190-200 ° C. After cooling the autoclave to 50 ° C, the excess of isobutylene was released and the reaction mixture was transferred with 1.5 l toluene into a 6 l flask. The solvent was evaporated under vacuum to a red oil. This residue was dissolved in 300 ml ethylacetate and the stirred solution was treated at rt (room temperature) over 2 hrs with 6 l hexane. After stirring overnight, the precipitate was filtered, washed with hexane and dried under vacuum at 35 ° C to 1308.8 g (83%) colourless crystals of 2-isobutenylsuccinic anhydride, m.p. 42-43 ° C, GC purity: 93%. This material was used directly in the next step.

The mother liquor was evaporated under vacuum and gave 132 g of a yellow oil which was triturated with 500 ml hexane and the slurry was stirred overnight at rt. The crystalline product was filtered, washed with hexane and dried under vacuum to 110.1 g (7%) slightly yellow crystals of 2-isobutenylsuccinic anhydride, GC-purity: 72%.

On a 100 g scale, this product was isolated in 93% yield and purity of 95%.

Example 2

A) In a 20 l flask 1418 g of 2-isobutenylsuccinic anliydride were dissolved in 141 ethylacetate and after addition of 140 g 5%-Pd on charcoal, degassing and establishing an internal hydrogen pressure of 1.1 bar, the reaction mixture was stirred for 5 hrs at rt. Alter flushing with an inert atmosphere, the liquid was filtered, transferred in a 25 l flask and the vessel was rinsed with ethylacetate. The solvent was evaporated under reduced pressure and the oily residue was dried at 40 ° C under high vacuum to 1534.9 g of crude isobutylsuccinic anhydride as a slightly yellow oil which was used directly in the next step. Purity according to GC: 98%, without internal standard.

B) In a 6 l flask, 767 g of the product of A) were dissolved in a mixture of 2.5 l ethanol and 1.25 l toluene. After the addition of 70 g p-toluenesulfonic acid, the solution was subjected to azeotropic distillation at 80 ° C. The residue was washed with sat. $NaHCO_3$-solution and the aqueous phase was back extracted with toluene. The org. phases were dried and filtered. The filtrate was distilled under reduced pressure to yield 837.7 g (79%) of rac-diethyl-isobutylsuccinate as a colourless liquid. Purity according to GC: 96%, without internal standard, b.p. 140 ° C/20 Torr or 68 ° C/0.1 Torr. This material was used directly in the next step.

In analogous expenments, a yield of 83% was achieved on a 3 kg scale. The purity varied from 96 to 98.5 GC-%.

Example 3

A) 3253 g of the product of Example 2B) were emulsified under stirring in a solution of 3.28 g sodium bicarbonate in 13.1 l water and the pH of the emulsion was adjusted to 8.0 using 2.0N NaOH. After addition of 160 ml Solvay Protease L 660, the reaction mixture was stirred at rt while maintaining the pH at 8.0 by addition of 2.0N NaOH-solution.

After 5 days, the reaction mixture was extracted with ethyl acetate. The organic phases were dried and concentrated to give 1615 g of (S)-diethylisobutylsuccinate as an oil. The aqueous phase was

acidified to pH 2.0 with 25% hydrochloric acid and extracted with ethyl acetate. After addition of sodium chloride, the aqueous phase was extracted again with ethyl acetate, the organic phases were dried and filtered. The filtrate was concentrated under reduced pressure and the residue was dried under high vacuum to 1352 g of (2R)-4-methyl-2-(ethoxycarbonylmethyl)valeric acid (47.3%) as a slightly brownish oil which was used directly in the next step. GC-purity: 96%, $[\alpha]_D^{25}$ = +19.1° (c=1%, ethanol).

B) 8.6 g sodium were dissolved in 250 ml ethanol and the solution was added at rt to 1720.4 g of (S)-diethyl-isobutylsuccinate. The solution was distilled under reduced pressure to yield 1542 g of rac.-diethyl-isobutylsuccinate (89.7%) as a colourless liquid. Purity according to GC: 94%, without internal standard. $[\alpha]_D^{25}$ = 0° (c=1, ethanol).

Example 4

A) 171.9 g of (2R)-4-methyl-2-(ethoxycarbonylmethyl)valeric acid were dissolved in 200 ml ethylacetate. After addition of 0.15 ml DMF, the solution was treated at rt with 124 ml thionylchloride. The reaction mixture was stirred at rt for 3.5 hrs and then evaporated under reduced pressure to 215 g of 2(R)-4-methyl-2-(ethoxycarbonylmethyl)valeric acid chloride as a slightly yellow oil which was used directly in the next step. Purity according to GC: 76%, without internal standard. $[\alpha]_D^{25}$ = +17.5° (c=1, methanol).

B) 194 g of the product of A) were dissolved in 800 ml THF. To this solution, 103.1 g of (S)-tert.butylglycine methylamide were added over 10-20 min while the reaction temperature was kept at 30°C. To this solution, cooled then to 15°C, were added 103.1 g triethylamine and the reaction mixture was stirred at rt for 5.5 hrs.

After quenching the reaction mixture in 700 ml water, the aqueous phase was extracted with ethylacetate. The org. phases were washed with 0.5N HCl and then with half sat.-$NaHCO_3$-solution. The filtrate was dried, filtered and the solvent evaporated under reduced pressure. The residue was dried under high vacuum at 40°C to give 207.5 g of N-2-[2(R)-(ethoxycarbonylmethyl)-4-methylvaleryl]-N-1,3-dimethyl-L-valinamide (91.6%) as slightly yellow crystals. Purity according to HPLC: 86.2%, with internal standard. This material was used directly in the next step. $[\alpha]_D^{25}$ = -6.3° (c=1, methanol), m.p. 102-103°C (from pentane).

Example 5

89.8 g KOH were dissolved at rt in 300 ml water and this solution was added to a solution of 55.6 g hydroxylamine hydrochloride in 100 ml water and stirred for 10 min. This solution was added to a stirred solution, cooled to 0-2°C, of 73.2 g of N-2-[2(R)-(ethoxycarbonylmethyl)-4-methylvaleryl]-N-1,3-dimethyl-L-valinamide in 150 ml THF and the reaction emulsion was stirred at 0°C for 3.75 hrs. After acidification of the reaction solution at 0°C to pH=7 with 2N HCl and stirring for additional 2 hrs at 0°C, the product began to precipitate and was filtered. The crystals were washed with water and dried under high vacuum to give 59.95 g of N-2-[2(R)-(hydroxycarbamoylmethyl)-4-methylvaleryl]-N-1,3-dimethyl-L-valinamide (99% yield) as colourless crystals, m.p. 206-207°C, HPLC purity: 93.1% (with internal standard).

## Claims

**1.** A process for the manufacture of carboxamides of the formula I:

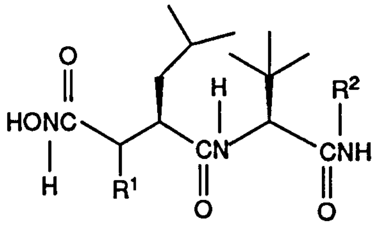

I

wherein

$R^1$ is H, protected amino, acylamino or lower alkyl optionally substituted by aryl, protected hydroxy, protected amino, acylamino, succinimido, 2,3-dihydro-1,3-dioxo-1H-benz[d,e]-isoquinol-2-yl, protected carboxy, carbamoyl, carboxy-lower alkanoylamino, pyrrolidino or

morpholino, and

$R^2$ is lower alkyl optionally substituted by protected carboxy or by carbamoyl, mono or di(lower alkyl)carbamoyl or morpholino,

which process comprises reacting a compound of the formula II:

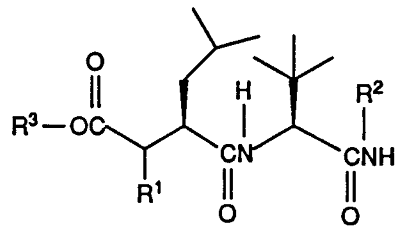

II

wherein $R^3$ is lower alkyl or aryl-lower alkyl, provided $R^3$ is not of the tert.butyl or neopentyl type, with a compound of the formula $H_2NO$-M, wherein M is an alkali metal.

**2.** A process according to claim 1 for the manufacture of a compound of the formula I-A

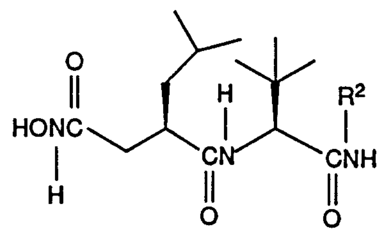

I-A

wherein $R^2$ is as in claim 1,
which process comprises reacting a compound of the formula II-A:

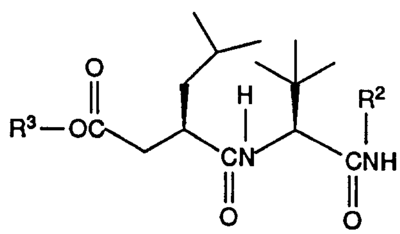

II-A

with a compound of the formula $H_2NO$-M, wherein M is an alkali metal.

**3.** A process for the manufacture of a compound of the formula II-A:

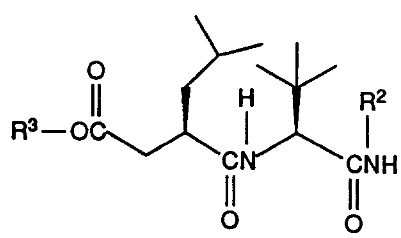

II-A

wherein $R^2$ is lower alkyl, optionally substituted by protected carboxy or by carbamoyl, mono or di-(lower alkyl)carbamoyl or morpholino, and $R^3$ is lower alkyl or aryl-lower alkyl, provided $R^3$ is not of the tert.butyl or neopentyl type,
which process comprises reacting a compound of the formula VI:

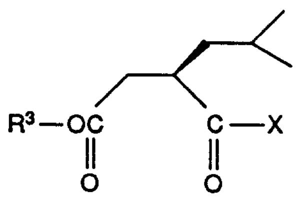

VI

wherein X is halogen, acyloxy or lower alkoxycarbonyloxy, with an amine of the formula VII:

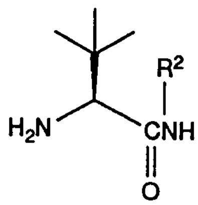

VII

wherein $R^2$ is as above.

**4.** A process for the manufacture of an acid of the formula III:

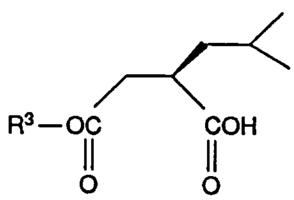

III

wherein $R^3$ is lower alkyl or aryl-lower alkyl, provided $R^3$ is not of the tert.butyl or neopentyl type, which process comprises enzymatically hydrolyzing a diester of the formula

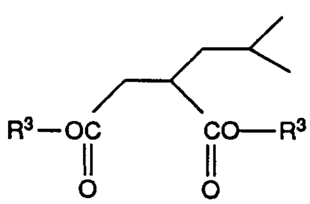

IV

wherein $R^3$ is as above.

**5.** A process for the manufacture of a diester of the formula IV

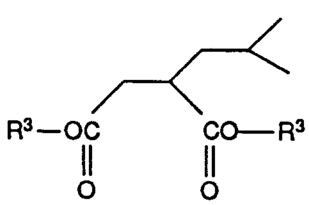

IV

wherein $R^3$ is lower alkyl or aryl-lower alkyl, provided $R^3$ is not of the tert.butyl or neopentyl type, which process comprises a) reacting maleic anhydride with isobutylene, b) hydrogenating the olefinic bound in the obtained compound of the formula

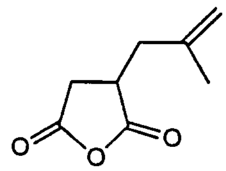

5

and c) reacting the obtained isobutylsuccinic anhydride with an alcohol of the formula $R^3$-OH.

**6.** A process according to claim 2 for the manufacture of a compound of the formula I-A:

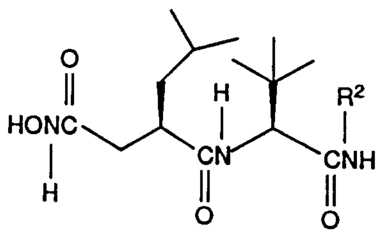

I-A

which process comprises a) converting an acid of the formula III:

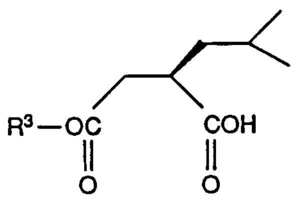

III

into an acid halogenide or into a mixed anhydride of the formula VI:

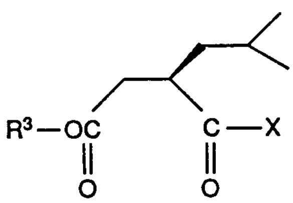

VI

b) reacting the compound of formula VI with an amine of the formula VII:

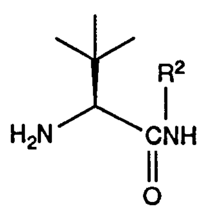

VII

and c) reacting the obtained compound of the formula II-A:

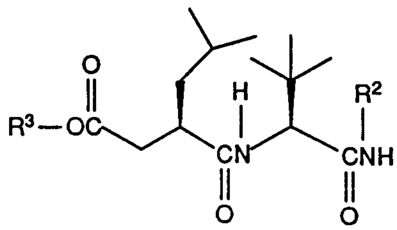

II-A

with a salt of the formula $H_2NO$-M, wherein M is an alkali metal.

**7.** A process according to claim 4, for preparing an acid of the formula III:

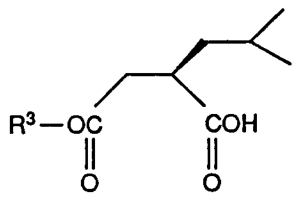

III

which process comprises a) reacting maleic anhydride with isobutylene, b) hydrogenating the olefinic bound in the obtained compound of the formula 5:

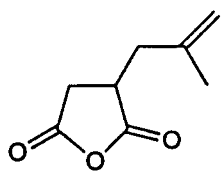

5

c) reacting the obtained isobutyl succinic anhydride with an alcohol of the formula $R^3$-OH, and d) enzymatically hydrolyzing the obtained diester of the formula IV:

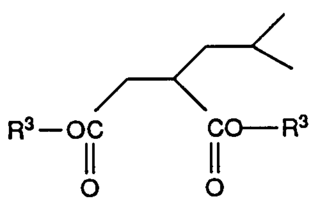

IV

**8.** The compound of the formulae II, II-A, VI and VIII

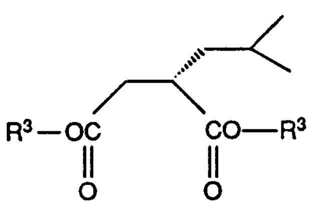

VIII

particularly the following:
N-2-[2(R)-(ethoxycarbonylmethyl)-4-methylvaleryl]-N-1,3-dimethyl-L-valinamide,
2(R)-4-methyl-2-(ethoxycarbonylmethyl)valeric acid chloride, and

(S)-diethyl-isobutylsuccinate.

European Patent Office

# EUROPEAN SEARCH REPORT

Application Number
EP 95 10 0292

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A,D | EP-A-0 497 192 (F. HOFFMANN-LA ROCHE AG)<br>* claims *<br>--- | 1-8 | C07C249/08<br>C07C237/22<br>C07C69/40 |
| X | TETRAHEDRON, (INCL. TETRAHEDRON REPORTS), vol.31, no.20, October 1975, OXFORD GB<br>pages 2602 - 2606<br>O. KORVER 'Chiroptical properties and conformation of some 2-alkyl substituted dicarboxylic acids.'<br>* page 2604; example 6; table 2 *<br>----- | 8 | |
| | | | TECHNICAL FIELDS SEARCHED (Int.Cl.6) |
| | | | C07C |

The present search report has been drawn up for all claims

| Place of search | Date of completion of the search | Examiner |
|---|---|---|
| THE HAGUE | 28 February 1995 | Sánchez García, J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document